# EUROPEAN PATENT SPECIFICATION

(11) **EP 3 752 954 B1**
(45) Date of publication and mention of the grant of the patent: **17.09.2025**
(21) Application number: 19707069.1
(22) Date of filing: 13.02.2019
(51) Int. Cl.: G06V 10/145, G06V 40/13

(54) **SKINPRINT ANALYSIS METHOD AND APPARATUS**
VERFAHREN UND VORRICHTUNG ZUR ANALYSE EINES HAUTABDRUCKS
PROCÉDÉ ET APPAREIL D'ANALYSE D'EMPREINTE CUTANÉE

(30) Priority: 13.02.2018 GB 201802357
(43) Date of publication of application: 23.12.2020
(73) Proprietor: Intelligent Fingerprinting Limited, Cambridgeshire CB24 9NG (GB)
(72) Inventor: WILSON, Paul, Cambridgeshire CB24 9NG (GB); GORDON, Benjamin, Cambridgeshire CB24 9NG (GB); HUDSON, Mark, Cambridgeshire CB24 9NG (GB); WALKER, Jeremy Nigel Burgess, Cambridgeshire CB24 9NG (GB)
(74) Representative: Boult Wade Tennant LLP
(86) International application number: PCT/GB2019/050386
(87) International publication number: WO 2019/158919

(56) References cited:
- EP-A2- 0 194 783
- WO-A1-2016/135497
- WO-A1-2018/029482
- WO-A2-2006/073450
- US-A1- 2002 050 713
- US-A1- 2012 127 128
- US-A1- 2013 285 977

## Description

### Background

An impression left by the friction ridges of human skin, such as the skin of a human finger, contains information regarding the identity of the human. It is widely known that the appearance of the impression of the human finger, known as a fingerprint, is unique to each human and may be used to confirm the identity of the human. The appearance of the impression of the skin of other human body parts may also be unique to each human and so may also be used to confirm the identity of the human. Impressions of human skin, including but not limited to the skin of the human finger, may be called skinprints.

In addition to the appearance of the impression left by human skin, the impression may contain chemical species which themselves may be detected in order to obtain further information. Skinprints comprise not only eccrine sweat but also may contain other constituents that may form a target for a diagnostic test. The Applicant has developed a range of techniques for detecting the presence of one or more analytes in skinprints.

For example, when a human intakes a substance (e.g. by ingestion, inhalation or injection) the substance may be metabolised by the human body giving rise to secondary substances known as metabolites. The presence of a particular metabolite can be indicative of a specific intake substance. The intake substance and/or metabolites may be present in sweat and, as such, may be left behind in a skinprint, e.g. a fingerprint. Detection of such metabolites in a skinprint can be used as a non-invasive method of testing for recent lifestyle activity such as (but not limited to) drug use, or compliance with a pharmaceutical or therapeutic treatment regime.

Importantly, the taking of a skinprint is much simpler than obtaining other body fluids such as blood, saliva and urine, and is more feasible in a wider range of situations. Not only this but since the appearance of the skinprint itself provides confirmation of the identity of the person providing the skinprint, there can be greater certainty that the substance or substances in the skinprint are associated with the individual. This is because substitution of a skinprint, particularly a fingerprint, is immediately identifiable from appearance whereas substitution of, for example, urine, is not immediately identifiable from appearance. As such, testing for one or more substances in a skinprint provides a direct link between the one or more substances and the identity of the human providing the skinprint.

The applicant has demonstrated various techniques for chemical analysis of skinprints deposited on a substrate (that is latent/residual skinprints) including the use of mass spectrometry, for example paper spray mass spectrometry. The applicant has also developed a lateral flow skinprint analysis technique as described in WO 2016/012812, published 28 January 2016. Document WO2018029482A1, which is prior art under Art. 54(3) EPC, discloses a method of determining presence of a skinprint using an apparatus comprising: a primary electromagnetic radiation source; an electromagnetic radiation detector; and a translucent waveguide comprising a first surface providing a waveguide interface coincident with a skinprint receiving region; the method comprising: transmitting primary electromagnetic radiation from the primary electromagnetic radiation source towards the waveguide interface at an angle of incidence relative to and on a first side of a normal line that is perpendicular to the waveguide interface, such that: (a) where the waveguide interface interfaces directly with ambient, the primary electromagnetic radiation incident on the waveguide interface reflects in the waveguide interface at an angle of reflection relative to and on a second side of the normal line opposite the first side; and (b) where a skinprint is present on the skinprint receiving region such that the waveguide interface interfaces with the skinprint and the skinprint interfaces with ambient, at least a portion of the primary electromagnetic radiation incident on the waveguide interface is transmitted through the waveguide interface into the skinprint; and using the electromagnetic radiation detector to determine an amount of primary electromagnetic radiation transmitted through the waveguide interface and/or reflected by the waveguide interface.

Document EP0194783 A2 discloses the influence of residual skin-print in the illumination angle of a fingerprint sensor using visible light.

Obtaining an indication of a quantity, for example mass (or possibly volume), of a metabolite present in a latent skinprint sample may be more informative if given as a measure relative to quantity, for example by volume or possibly by mass), of skinprint. This may be particularly applicable in situations where an acceptable threshold (measured in, for example, mass of analyte per unit volume of skin-print) is defined, for example by an independent standards agency.

For example, a relatively small amount of metabolite present in a relatively large volume/mass of skinprint may be less significant than a relatively larger amount of metabolite present in only a relatively small volume/mass of skinprint.

Accordingly, a need exists for a technique to determine a quantity of skinprint deposited in order to be able to provide an indication of an amount of analyte per unit of deposited skinprint.

It is known to use a quartz crystal microbalance to measure small mass increments. This technique does not lend itself well to robust in-the-field determination of fingerprint or skinprint mass measurement. Furthermore, since the quantity of constituents in a deposited skinprint is modest, a very precise balance is necessary. Alternative approaches to measuring a total quantity of skinprint include the following:
Interferometry;
White light interferometry;
Detecting the influence on passage of electromagnetic radiation;
Surface plasmon resonance imaging;
Optical imaging and software processing;
Optical coherence tomography;
Confocal microscopy;
Atomic force microscopy;
3D laser mapping;
Ellipsometry;
Scanning tunnelling microscopy;
Image analysis following staining with a developer agent such as Ninhydrin; and
Image analysis following staining with a detection agent such as Nile red.

The Applicant has identified a need for a technique that is both accurate and cost-effective for determining volume of a skinprint deposited on a surface. Minimising time taken, so as to facilitate a high-throughput process, is also desirable. Also desirable is an apparatus for carrying out the method that is compact and portable.

### Summary of the disclosure

Against this background, there is provided a method of determining volume of a deposited residual skinprint, the method comprising:
using an apparatus comprising: a primary electromagnetic radiation source configured to emit visible spectrum electromagnetic radiation; an electromagnetic radiation detector; and a translucent waveguide comprising a first surface providing a waveguide interface coincident with a skinprint receiving region;
transmitting primary electromagnetic radiation from the primary electromagnetic radiation source towards the waveguide interface at an angle of incidence relative to and on a first side of a normal line that is perpendicular to the waveguide interface, such that:
   (a) where the waveguide interface interfaces directly with ambient, the primary electromagnetic radiation incident on the waveguide interface undergoes total internal reflection so as to reflect in the waveguide interface at an angle of reflection relative to and on a second side of the normal line opposite the first side; and
   (b) where a deposited skinprint is present on the skinprint receiving region such that the waveguide interface interfaces with the skinprint and the skinprint interfaces with ambient, at least a portion of the primary electromagnetic radiation incident on the waveguide interface is caused by the skinprint to be transmitted through the waveguide interface;
using the electromagnetic radiation detector to determine an amount of primary electromagnetic radiation transmitted through the waveguide interface or reflected by the waveguide interface; and
using calibration data to equate the amount of primary radiation transmitted through the waveguide interface and/or reflected by the waveguide interface to the volume of skinprint deposited on the substrate.

In this way, a skinprint may be used to couple electromagnetic radiation into or out of a translucent waveguide. The extent of the coupled electromagnetic radiation is detected and compared with calibration data in order to provide an indication of the volume of the deposited skinprint, within a small and defined level of uncertainty and facilitates determination of concentration of analytes in the skin-print.

Embodiments of the disclosure will now be described, by way of example only, with reference to the accompanying drawing in which:

### Brief description of the drawings

Figure 1a provides a schematic representation of a first embodiment of the disclosure showing behaviour of electromagnetic radiation in the event that a skinprint is present;
Figure 1b provides a schematic representation of the first embodiment of the disclosure showing behaviour of electromagnetic radiation in the event that no skinprint is present;
Figure 2a provides a schematic representation of a second embodiment of the disclosure showing behaviour of electromagnetic radiation in the event that a skinprint is present;
Figure 2b provides a schematic representation of the second embodiment of the disclosure showing behaviour of electromagnetic radiation in the event that no skinprint is present;
Figure 3a provides a schematic representation of a third embodiment of the disclosure showing behaviour of electromagnetic radiation in the event that a skinprint is present;
Figure 3b provides a schematic representation of the third embodiment of the disclosure showing behaviour of electromagnetic radiation in the event that no skinprint is present;
Figure 4a provides a schematic representation of a fourth embodiment of the disclosure showing behaviour of electromagnetic radiation in the event that a skinprint is present;
Figure 4b provides a schematic representation of the fourth embodiment of the disclosure showing behaviour of electromagnetic radiation in the event that no skinprint is present;
Figure 5a provides a schematic representation of a fifth embodiment of the disclosure showing behaviour of electromagnetic radiation in the event that a skinprint is present;
Figure 5b provides a schematic representation of the fifth embodiment of the disclosure showing behaviour of electromagnetic radiation in the event that no skinprint is present;
Figure 6a provides a schematic representation of a sixth embodiment of the disclosure showing behaviour of electromagnetic radiation in the event that a skinprint is present;
Figure 6b provides a schematic representation of the sixth embodiment of the disclosure showing behaviour of electromagnetic radiation in the event that no skinprint is present;
Figure 7a provides a schematic representation of a seventh embodiment of the disclosure showing behaviour of electromagnetic radiation in the event that a skinprint is present;
Figure 7b provides a schematic representation of the seventh embodiment of the disclosure showing behaviour of electromagnetic radiation in the event that no skinprint is present;
Figure 8a provides a schematic representation of an eighth embodiment of the disclosure showing behaviour of electromagnetic radiation in the event that a skinprint is present;
Figure 8b provides a schematic representation of the eighth embodiment of the disclosure showing behaviour of electromagnetic radiation in the event that no skinprint is present;
Figure 9a provides a schematic representation of a ninth embodiment of the disclosure showing behaviour of electromagnetic radiation from a primary source in the event that a skinprint is present;
Figure 9b provides a schematic representation of the ninth embodiment of the disclosure showing behaviour of electromagnetic radiation from the primary source in the event that no skinprint is present;
Figure 9c provides a schematic representation of the ninth embodiment of the disclosure showing behaviour of electromagnetic radiation from a secondary source regardless of whether or not a skinprint is present; and
Figure 10 shows a plot of the relationship between volume of skinprint as determined using an apparatus of the arrangement shown in Figures 9a, 9b and 9c and volume determined using a white light interferometry technique.

### Detailed description

The disclosure relates to a method and apparatus for determining volume of a skinprint 30 deposited on a surface. A wide range of alternative implementations is envisaged. The following detailed description relates to a subset of embodiments that fall within the scope of the appended claims.

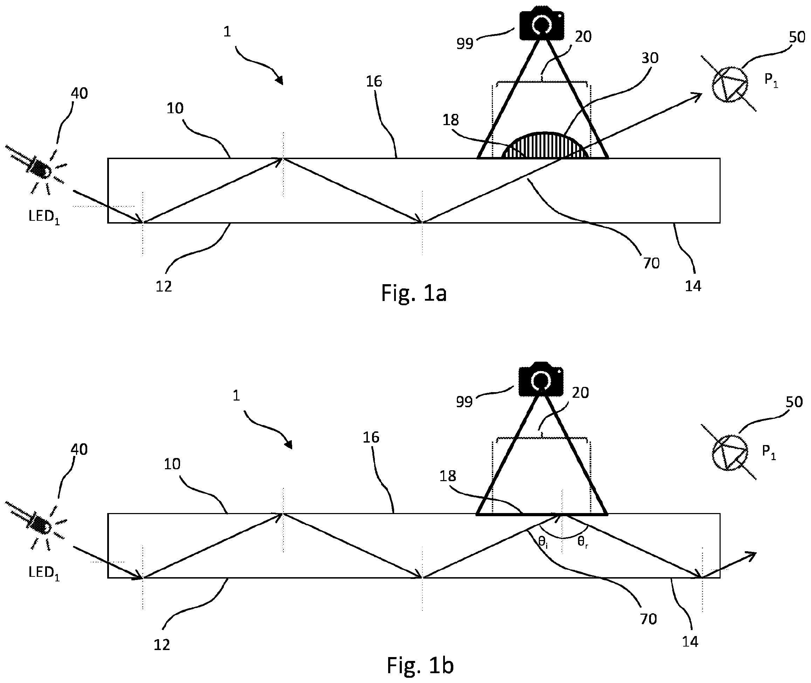

Figure 1a and 1b show a schematic representation of a first embodiment 1 of the disclosure. Figure 1a shows behaviour of electromagnetic radiation in the first embodiment 1 where a skinprint 30 is present while Figure 1b shows behaviour of electromagnetic radiation in the first embodiment where no skinprint is present. The first embodiment 1 comprises an LED 40, a photodiode 50 and a translucent waveguide 10 between the LED 40 and the photodiode 50 configured to output a photodiode signal indicative of electromagnetic radiation detected by the photodiode 50.

The translucent waveguide 10 comprises a first end 12 and a second end 14. The LED 40 is optically coupled to the translucent waveguide 10 towards the first end 12.

The second end 14 comprises a fingerprint receiving region 20 on a first surface 16 of the translucent waveguide 10. The fingerprint receiving region 20 may be identified on the first surface 16 by virtue of one or more visible indications on or surrounding the fingerprint receiving region 20. Alternatively, the fingerprint receiving region 20 may be identified by a window bounded by a frame that obscures parts of the first surface 16 that do not form part of the fingerprint receiving region 20. The fingerprint receiving region 20 may be identified by other means.

The fingerprint receiving region 20 may provide a fixed area onto which a skinprint may be applied in order to increase consistency of area of skinprints between donors. The fixed area may be smaller than the average skinprint area and/or smaller than an area of an average adult skinprint. This may also have advantages for consistency if the same subject provides multiple prints, perhaps for different purposes.

A surface of the translucent waveguide 10 in the vicinity of the fingerprint receiving region 20 may serve as a waveguide interface 18 through which electromagnetic radiation may be transmitted or in which electromagnetic radiation may be reflected, dependent upon circumstances. The waveguide interface 18 may or may not be different in surface properties when compared to a surface of the translucent waveguide 10 that surrounds the waveguide interface 18.

The photodiode 50 is located so as to detect electromagnetic radiation that is transmitted out of the translucent waveguide 10 via the waveguide interface 18.

The LED 40 is optically coupled to the translucent waveguide 10 towards the first end 12 such that electromagnetic radiation 70 emitted by the LED 40 enters into the translucent waveguide 10 at an angle such that the electromagnetic radiation 70 is retained within the translucent waveguide by total internal reflection. Optical coupling of the LED 40 to the translucent waveguide 10 may take any appropriate form. At the point of entry of the electromagnetic radiation 70 into the translucent waveguide 10, some refraction of the electromagnetic radiation 70 may take place. (For the sake of clarity, this refraction is not shown in the Figures.) In particular, electromagnetic radiation 70 that is incident upon an end surface of the translucent waveguide 10 at an angle of incidence is transmitted into the translucent waveguide 10 with a small change in direction away from a normal line (which is shown in the Figure) that is perpendicular to the surface through which the electromagnetic radiation 70 enters the translucent waveguide 10. The extent of the refraction that takes place depends upon the ratio between the index of refraction of the translucent waveguide 10 and the index of refraction of the material through which the electromagnetic radiation 70 travels immediately prior to reaching the point of entry. Where the material immediately prior to the electromagnetic radiation 70 reaching the point of entry is ambient air, the ratio is likely to be higher (and so the extent of the refraction is likely to be greater) than if the material immediately prior to the electromagnetic radiation 70 reaching the point of entry is, for example, a translucent encapsulation material of an LED package. Accordingly, the nature and extent of any refraction will depend upon how the electromagnetic radiation 70 is coupled from the electromagnetic radiation source 40 into the translucent waveguide 10.

Subsequently, as the electromagnetic radiation 70 travelling within the translucent waveguide 10 reaches the edges of the translucent waveguide 10, it arrives at an angle of incidence that is such as to cause the electromagnetic radiation 70 to reflect at the perimeter of the translucent waveguide 10 as a result of total internal reflection rather than to be transmitted out of the translucent waveguide 10. This pattern of total internal reflection is reproduced along the translucent waveguide 10 and by this mechanism the electromagnetic radiation 70 propagates along and within the translucent waveguide 10.

While in Figure 1a a skinprint 30 is shown in situ on the skinprint receiving region 20, in Figure 1b no skinprint is present on the skinprint receiving region 20. A comparison between Figures 1a and 1b illustrates how behaviour of electromagnetic radiation 70 is influenced by the presence or absence of a skinprint 30 on the skinprint receiving region. Not only is it the case that presence or absence of a skinprint influences electromagnetic radiation 70 in this way, but the Applicant has found that the volume of skinprint material deposited corresponds closely to the extent of the influence of the skinprint on the electromagnetic radiation.

In the case that no skinprint is present on the skinprint receiving region, as is evident from Figure 1b, a further total internal reflection occurs at the location of the skinprint receiving region 20 such that the electromagnetic radiation 70 continues to propagate along the translucent waveguide 10. When the electromagnetic radiation 70 reaches the end of the translucent waveguide 10, it arrives at an angle such that it passes through the end of the translucent waveguide 10, albeit undergoing some refraction (again for the sake of clarity not shown in Figure 1b) and thereby exits the translucent waveguide 10.

By contrast, as can be seen from Figure 1a, in the case that a skinprint 30 is present on the skinprint receiving region 20, at least a portion of the electromagnetic radiation 70 that arrives at the skinprint receiving region 20 is transmitted out of the translucent waveguide 10 at the waveguide interface 18 by virtue of the presence of the skinprint 30. This is because the waveguide interface 18 is (at least partially) covered by residue of the constituents of the skinprint, hereafter for brevity referred to simply as the skinprint 30. Therefore, instead of the waveguide interface 18 interfacing directly with ambient conditions (such as ambient air) wherein the difference in refractive indices between the translucent waveguide 10 and ambient would be such as to result in total internal reflection, the waveguide interface 18 interfaces directly with the skinprint 30. The ratio of refractive indices between that for the translucent waveguide 10 and that for the skinprint 30 is such that at least some of the electromagnetic radiation 70 is transmitted through the waveguide interface 18 and into the skinprint. When the electromagnetic radiation 70 reaches the surface of the skinprint (opposite the translucent waveguide 10) a combination of the ratio of refractive indices between that for the skinprint 30 and that for the ambient together with the angle of incidence of the electromagnetic radiation 70 at the interface results in at least some of the electromagnetic radiation 70 being transmitted out of the skinprint 30.

In the embodiment of Figures 1a and 1b, electromagnetic radiation 70 that is transmitted via the waveguide interface 18 and out of the skinprint 30 is received at the photodiode 50. In very general terms, the greater the volume of the skinprint, the more electromagnetic radiation 70 is received by the photodiode 50. Accordingly, there is a relationship between the volume of deposited skinprint 30 on the skinprint receiving region 20 and the amount of electromagnetic radiation 70 detected by the photodiode 50. Where no skinprint is present, little or no electromagnetic radiation 70 will be detected by the photodiode 50 because it remains within the translucent waveguide 10. Where a well-defined, strong skinprint is present, a significant proportion of the electromagnetic radiation 70 will be coupled out of the waveguide interface and will reach the photodiode 50.

A discussion of the correspondence between electromagnetic radiation 70 received by the photodiode 50 and volume of skinprint as determined by a white light interferometer technique is provided below.

It should be noted that Figures 1a and 1b (as well as the corresponding Figures relating to other embodiments) are highly schematic. As the skilled person would readily understand, the analysis is not binary. That is to say, it is not the case that in the event of a skinprint 30 being present all electromagnetic radiation 70 will transmit out of the translucent waveguide 10 via the waveguide interface 18. Similarly, it is not the case that in the event of no skinprint is present, no electromagnetic radiation 70 will transmit out of the translucent waveguide via the waveguide interface 18. In reality, some electromagnetic radiation 70 will transmit out of the translucent waveguide when no skinprint 30 is present. Conversely, when a skinprint 30 is present some electromagnetic radiation 70 will remain in the translucent waveguide.

Furthermore, it should be noted that the electromagnetic radiation 70 will not all travel in exactly the directions indicated by the arrows in Figures 1a and 1b. In short, Figures 1a and 1b are schematic and are intended to illustrate the principles.

In the Figures, the schematic representation of a skinprint 30 (where present) is such as to suggest that it is manifested as a single dome-shaped form on the skinprint receiving region 20. It is emphasised that this representation is highly schematic. Again as the skilled person readily appreciates, the form of skinprints varies significantly depending upon many factors including the amount of eccrine sweat on the surface of the skin when printed and the force with which a user places the skin against the skinprint receiving region 20 when providing a skinprint. In reality, the skinprint is likely to comprise a number of peaks and troughs, all of which may influence the behaviour of electromagnetic radiation incident upon it in a variety of ways. The peaks may contain sebaceous sweat as well as eccrine sweat which may differently influence the behaviour of the electromagnetic radiation.

As can be seen from Figures 1a and 1b, the first embodiment may further comprise optical imaging capability, as illustrated schematically by a camera icon 99. The optical imaging capability may be employed to provide an optical image of the skinprint that might be compared with a database of skinprint images, so as to confirm identity of a skinprint. The optical image functionality is equally applicable to any of the other embodiments disclosed herein but, for the sake of clarity, it is not illustrated other than in Figures 1a and 1b.

The applicant has developed various techniques for chemical analysis of skinprints. In order to determine that the chemical analysis is feasible for a given skinprint, it is helpful to have an indication that there is sufficient material present in a skinprint in order to apply a particular chemical test and, in particular, to quantify results of the chemical analysis relative to a mass or volume of the skinprint under test. The techniques described herein provide a measure of the volume of skinprint that has been deposited on the skinprint receiving region.

The apparatus of the first embodiment may comprise controller circuitry configured to receive the photodiode signal and process that signal in order to determine whether a skinprint volume threshold is met. The controller may, for example, be configured to receive a first (reference) photodiode signal prior to a user providing a skinprint on the skinprint receiving region and to receive a second photodiode signal once a skinprint has been provided on the skinprint receiving region and to compare the first and second signals.

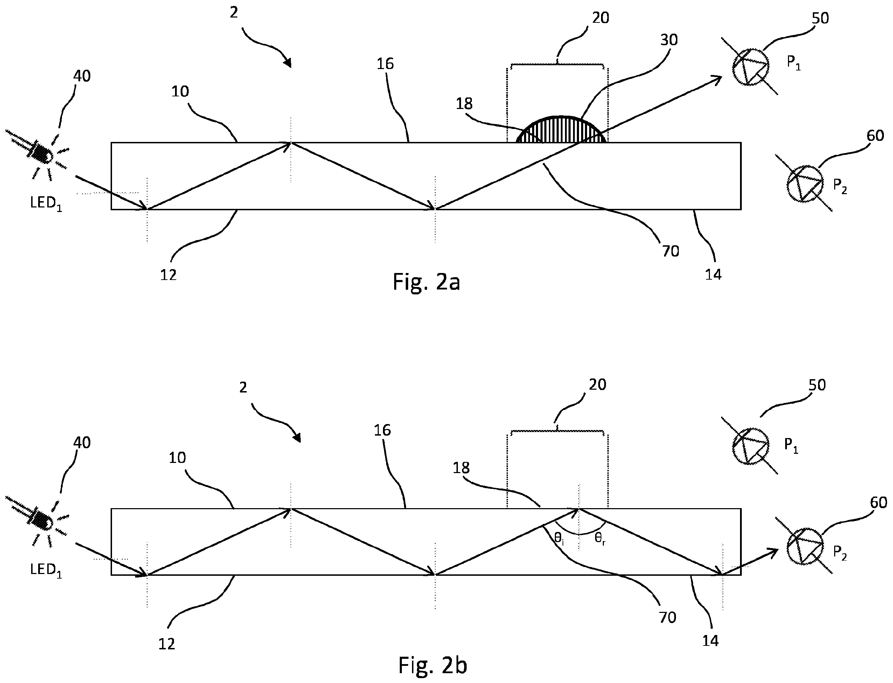

Figures 2a and 2b show a second embodiment 2 of the disclosure. The second embodiment 2 of the disclosure differs from the first embodiment in that a second photodiode 60 is provided in addition to the first photodiode 50. The second photodiode 60 is intended to detect electromagnetic radiation 70 that is not transmitted through the waveguide interface and is instead propagated by total internal reflection throughout the translucent waveguide 10. By providing two photodiodes and obtaining a signal from each indicative of an amount of electromagnetic radiation detected by each, the signals from each of the first and second photodiodes 50, 60 can be compared as part of a calculation to determine skinprint volume.

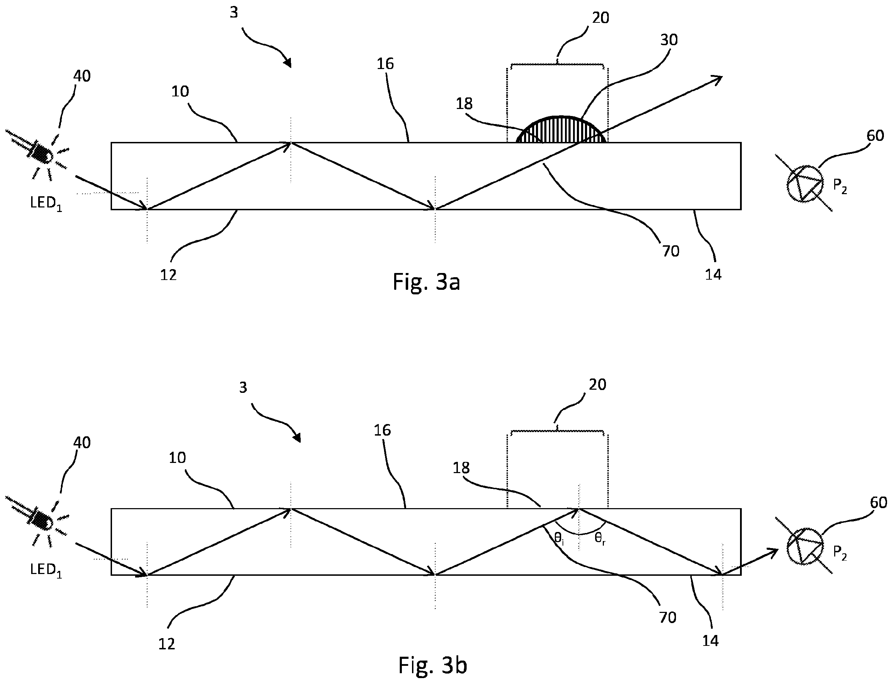

Figures 3a and 3b show a third embodiment 3 of the disclosure. The third embodiment 3 of the disclosure differs from the first and second embodiments 1, 2 in that only the second photodiode 60 (and not the first photodiode) is provided. In this way, the photodiode 60 only detects electromagnetic radiation 70 that is not transmitted through the waveguide interface and is instead propagated by total internal reflection throughout the translucent waveguide 10.

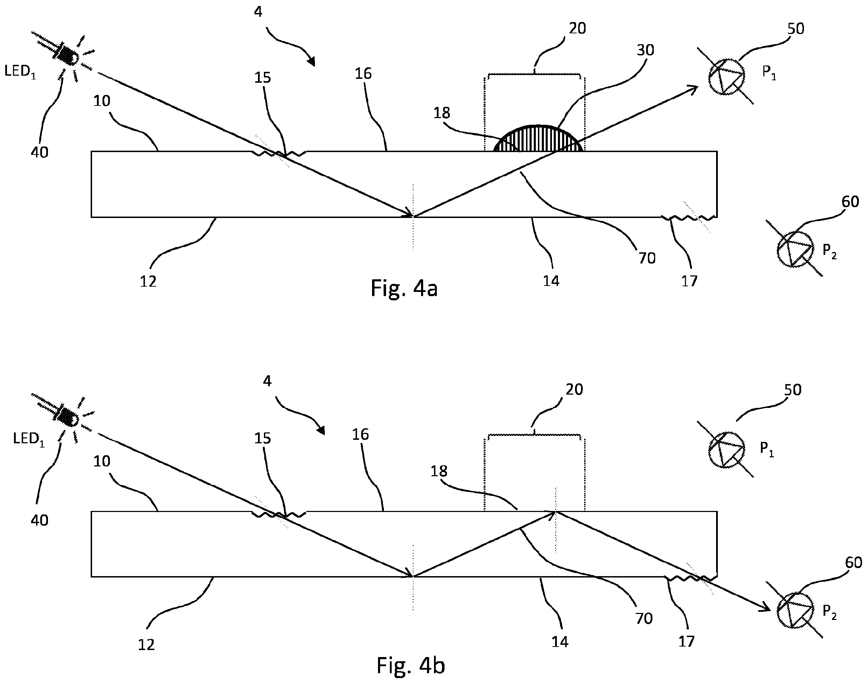

Figures 4a and 4b show a fourth embodiment 4 of the disclosure. The fourth embodiment 4 of the disclosure differs from the second embodiment 2 in that electromagnetic radiation 70 is transmitted (coupled) into the translucent waveguide 10 via a first grating coupler 15 and in that electromagnetic radiation 70 that is not transmitted out of the waveguide interface 18 and continues to propagate through the translucent waveguide 10 by total internal reflection is transmitted (coupled) out of the translucent waveguide 10 via a second grating coupler 17. The first grating coupler 15 may comprise a roughened portion of a surface of the translucent waveguide 10 through which electromagnetic radiation may pass into the translucent waveguide 10. This may provide flexibility regarding location of the LED 40 relative to the translucent waveguide 10. This may be particularly appropriate when providing the apparatus in a compact portable package. The second grating coupler 17 may comprise a roughened portion of a surface of the translucent waveguide 10 through which electromagnetic radiation may pass out of the translucent waveguide 10. This may provide flexibility regarding location of the second photodiode 60 relative to the translucent waveguide 10. Again, this may be particularly appropriate when providing the apparatus in a compact portable package.

As the skilled person would readily appreciate, alternative embodiments (not illustrated) may involve only one of the two grating couplers 15, 17. For example, an alternative embodiment may include a first grating coupler 15 in the absence of a second grating coupler 17. In such an embodiment electromagnetic radiation 70 that is not transmitted out of the waveguide interface 18 and continues to propagate through the translucent waveguide 10 by total internal reflection may be transmitted (coupled) out of the translucent waveguide 10 in the same manner as in the second and third embodiments 2, 3. Similarly, a further alternative embodiment may include a second grating coupler 17 in the absence of a first grating coupler 15. In such an embodiment, electromagnetic radiation 70 may be coupled into the translucent waveguide 10 in the same manner as for the first, second and third embodiments 1, 2, 3.

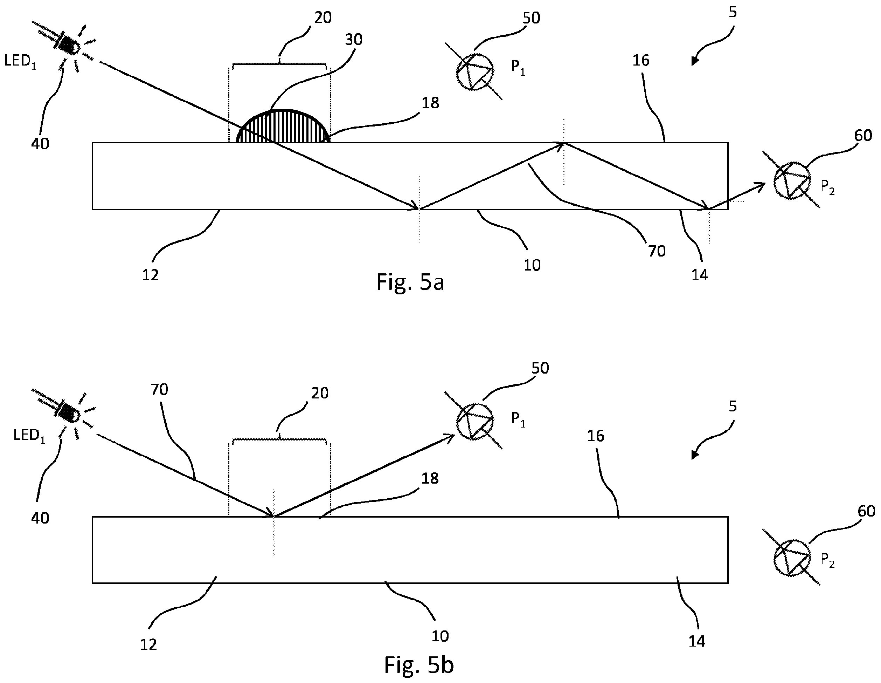

Figures 5a and 5b show a fifth embodiment 5 of the disclosure. The fifth embodiment 5 differs from the first to fourth embodiments 1, 2, 3, 4 in that the direction of potential transmission through the waveguide interface 18 (in the presence of a skinprint) is into the translucent waveguide 10 rather than out of the translucent waveguide 10. Accordingly, the electromagnetic radiation source 40 is located such that electromagnetic radiation 70 reaches the waveguide interface 18 from the exterior of the translucent waveguide 10 towards the first end 12 of the translucent waveguide 10. In addition, the fingerprint receiving region 20 is located on the first surface 16 of the translucent waveguide 10 also towards the first end 12 of the translucent waveguide 10. In the event that a skinprint is present, electromagnetic radiation 70 is transmitted through the waveguide interface 18 and into the translucent waveguide 10 for onward propagation towards the second end 14 of the translucent waveguide 14 through total internal reflection as shown schematically in Figure 5a. In the event that no skinprint is present, electromagnetic radiation simply reflects off the waveguide interface 18 and thereby never enters the translucent waveguide 10 as shown in Figure 5b. In the illustration, electromagnetic radiation that is reflected in the waveguide interface 18 is detectable by a first photodiode 50 and electromagnetic radiation that is transmitted through the waveguide interface 18 via a skinprint is detectable by a second photodiode 60. However, in common with the differences between the first, second and third embodiments 1, 2, 3, it may be appropriate to have only one rather than both of the photodiodes.

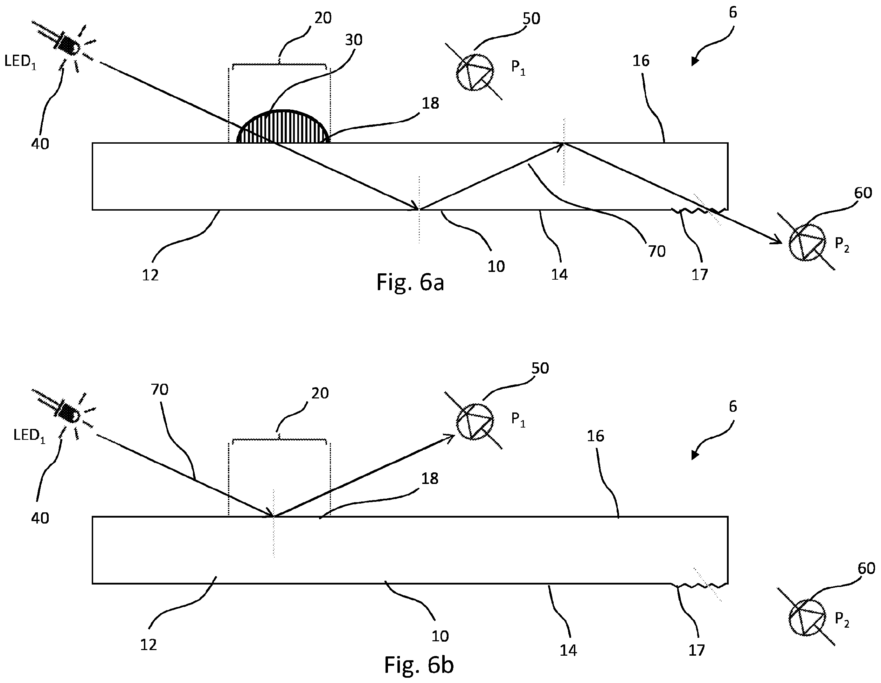

Figures 6a and 6b show a sixth embodiment 6 of the disclosure. The sixth embodiment 6 differs from the fifth embodiment 5 in that electromagnetic radiation 70 that is transmitted through the waveguide interface 18 via a skinprint 30 is transmitted out of the waveguide 10 via an output grating coupler 17, as described previously in relation to the fourth embodiment 4.

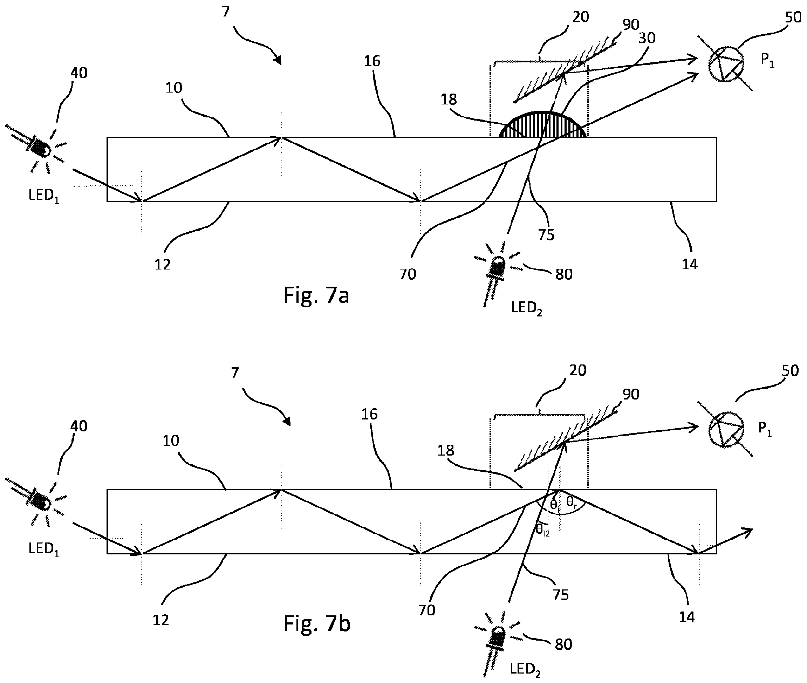

Figures 7a and 7b show a seventh embodiment 7 of the disclosure. The seventh embodiment 7 is similar to the first embodiment 1 and further comprises a secondary electromagnetic radiation source 80. The secondary electromagnetic radiation source 80 is located so that secondary radiation 75 emitted from the secondary electromagnetic radiation source 80 travels at an angle such that it transmits directly through the waveguide interface 18 whether or not a skinprint is present. Effectively, therefore, the secondary electromagnetic radiation acts as a reference to which the primary electromagnetic radiation is compared.

Accordingly, when no skinprint is present, only the secondary radiation 75 reaches the photodetector 50. This is because the primary electromagnetic radiation 70 from the primary electromagnetic radiation source 40 is reflected by the waveguide interface 18 rather than being transmitted through it. (A reflector 90 may be used to ensure that the secondary radiation, once out of the waveguide 10, is directed to the photodetector 50.) When a skinprint 30 is present, primary radiation 70 from the primary radiation source 40 passes through the waveguide interface 18 such that both primary and secondary radiation 70, 75 reach the photodetector 50.

In one aspect of the seventh embodiment 7, one or both of the primary and secondary radiation sources 40, 80 may be pulsed. For example, if the primary radiation source 40 is constant and the secondary radiation source 80 is pulsed then the primary radiation 70 can be detected when the secondary radiation source 80 is off. A value for the secondary radiation 80 can be calculated by subtracting the measured primary radiation 70 from the measured combination of primary and secondary radiation when the secondary radiation source 80 is on.

If the primary and secondary radiation sources 40, 80 are of the same specification (e.g. in terms of brightness and spectrum) then they will both be affected by the material properties of the translucent waveguide 10 in the same way. Accordingly, it is possible by this technique to eliminate variations that arise from the use of different waveguides. This may be particularly appropriate where the waveguide 10 is a consumable product that is replaced with each test performed.

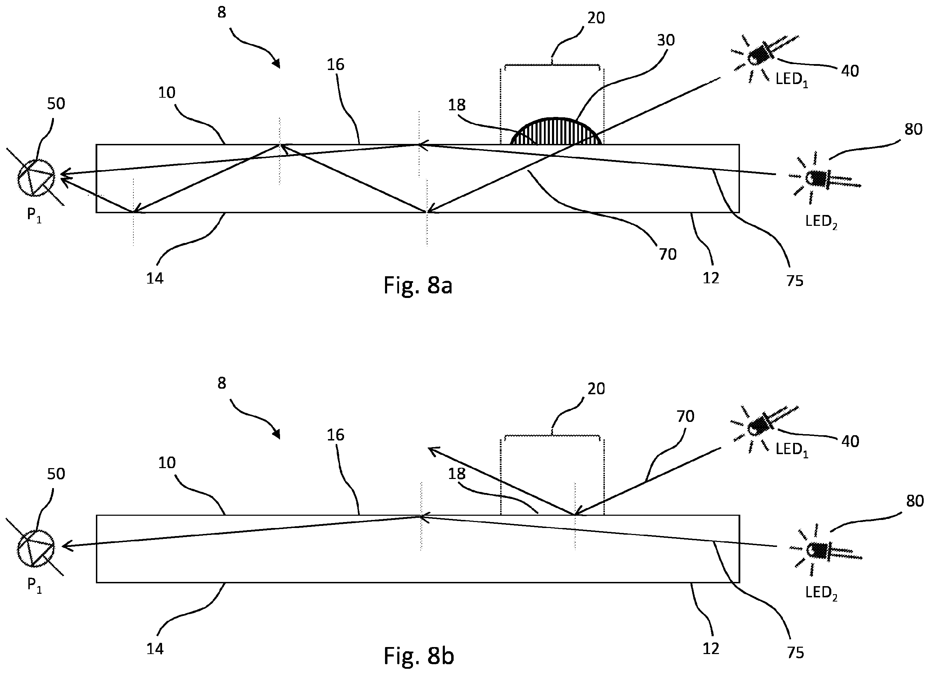

Figure 8a and 8b show an eighth embodiment of the disclosure. In common with the seventh embodiment 7, the eighth embodiment 8 comprises both primary and secondary electromagnetic radiation sources 40, 80. The primary and secondary electromagnetic radiation sources 40, 80 are both located towards a first end 12 of the translucent waveguide 10. The skinprint receiving region 20 is also located towards the first end 12 of the translucent waveguide 10. A photodetector 50 is located towards the second end 14 of the translucent waveguide 10.

In common with the fifth and sixth embodiments (and by contrast with the first, second, third, fourth and seventh embodiments), the direction of potential transmission through the waveguide interface 18 (in the presence of a skinprint) is into the translucent waveguide 10 rather than out of the translucent waveguide 10.

The primary electromagnetic radiation source 40 is located such that primary electromagnetic radiation 70 reaches the waveguide interface 18 from the exterior of the translucent waveguide 10 towards the first end 12 of the translucent waveguide 10. In addition, the fingerprint receiving region 20 is located on the first surface 16 of the translucent waveguide 10 also towards the first end 12 of the translucent waveguide 10. In the event that a skinprint is present, electromagnetic radiation 70 is transmitted through the waveguide interface 18 and into the translucent waveguide 10 for onward propagation towards the second end 14 of the translucent waveguide 14 through total internal reflection as shown schematically in Figure 8a. In the event that no skinprint is present, as shown in Figure 8b, primary electromagnetic radiation 70 from the primary electromagnetic radiation source 40 simply reflects off the waveguide interface 18 and does not enter the translucent waveguide 10.

The secondary electromagnetic radiation source 80 is located such that secondary radiation 75 is directed into the translucent waveguide 10 at an angle such that it propagates through the translucent waveguide 10 without opportunity for it to be coupled out of the translucent waveguide 10 until it reaches the second end 14 of the translucent waveguide in the region of the photodetector 50. This may be achieved by directing the secondary electromagnetic radiation 75 into the translucent waveguide 10 in a direction that is only marginally angled relative to the first surface 16 of the translucent waveguide 10 (or potentially substantially parallel to the first surface). In this way, the angle of travel of the secondary electromagnetic radiation 75 through the translucent waveguide 10 is such that neither the presence nor the absence of a skinprint 30 enables the radiation to be coupled out of the translucent waveguide 10, at least to any substantial degree.

Electromagnetic radiation (whether primary or secondary) that reaches the second end 14 of the translucent waveguide 10 is detected by the first photodiode 50. In the event that no skinprint 30 is present on the skinprint receiving region 20 (see Figure 8b), primary electromagnetic radiation 70 will not be coupled into the translucent waveguide 10 via the waveguide interface 18 and therefore only secondary radiation 75 will arrive at the photodiode 50. By contrast (see Figure 8a), in the event that a skinprint 30 is present on the skinprint receiving region 20, primary radiation 70 that is coupled into the translucent waveguide 10 via the waveguide interface 18 as a result of the presence of a skinprint 30, will arrive at the photodiode 50 in addition to secondary radiation 75.

As in the seventh embodiment, the secondary radiation 75 (resulting from the second electromagnetic radiation source 80) acts as a reference with which the primary radiation 70 (resulting from the primary electromagnetic radiation source 40) can be compared.

Secondary radiation 75 that is emitted by the second electromagnetic radiation source 80 but fails to reach the photodiode 50 is not attributable to skinprint volume but is instead attributable to properties of the substrate and the photodiode. By determining these losses, it is then possible to determine the extent to which such losses will also impact on the primary radiation 75. Accordingly, compensation can be made to account for such losses and thereby have greater confidence that the remaining difference is attributable to skinprint volume.

As in the seventh embodiment, one or both of the primary and secondary radiation sources 40, 80 may be pulsed. For example, if the primary radiation source 40 is constant and the secondary radiation source 80 is pulsed then the primary radiation 70 can be detected in isolation when the secondary radiation source 80 is off. Where no skinprint 30 is present (such that minimal primary radiation would be expected to arrive at the photodiode 50) the photodiode would detect radiation only when the secondary radiation source 80 is on.

Alternatively, the secondary radiation source 80 may be constant and the primary radiation source 40 may be pulsed. In this way, where no skinprint is present there should be little difference between the radiation detected by the photodetector 50 regardless of the pulsed nature of the primary radiation 70 since the primary radiation 70 (when on) is not coupled into the translucent waveguide 10 and therefore does not reach the photodetector 50.

If the primary and secondary radiation sources 40, 80 are of the same specification (e.g. in terms of brightness and spectrum) then they will both be affected by the material properties of the translucent waveguide 10 in the same way. Accordingly, it is possible by this technique to eliminate variations that arise from the use of different waveguides. This may be particularly appropriate where the waveguide 10 is a consumable product that is replaced with each test performed.

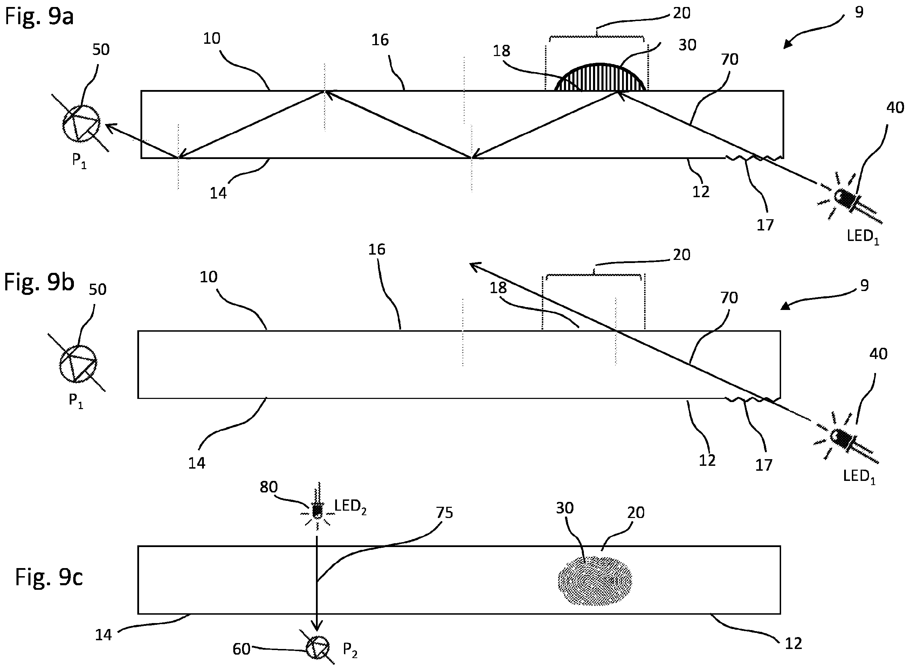

Figures 9a, 9b and 9c show a ninth embodiment of the disclosure. This embodiment uses primary and secondary radiation sources 40, 80. The primary radiation source 40 is configured to supply primary radiation 70 the underside of the translucent waveguide 10 at an angle such that skinprint elements on the skinprint receiving region 20 cause the radiation to be coupled out of the waveguide whereas the remaining primary radiation not incident on elements of skinprint is totally internally reflected so as to be retained within the translucent waveguide 10. Primary radiation that is totally internally reflected is detected by the first photodetector 50.

Separately, a secondary radiation source 80 is configured to supply secondary radiation 75 at a different location within the waveguide where it is immune to the presence or absence of a skinprint on the skinprint receiving region 20. A second photodetector 60 is configured to detect the secondary radiation 75.

In this way the secondary radiation 75 is unaffected by skinprint volume (or even presence) and provides optical reference data regarding the waveguide itself that can be used as a reference against which to compare the primary radiation 70 detected by the primary photodetector 50.

In any embodiment involving both primary and secondary radiation, as an alternative to the pulsing strategy for separating primary and secondary radiation detected at the photodetector 50, it may be possible to use primary radiation having a different colour from that of the secondary radiation and use a colour sensitive photodetector to distinguish between the primary and secondary radiation. In short, any appropriate technique for distinguishing between primary and secondary radiation may be employed. Such techniques may include separation in the frequency domain, separation in the time domain, and separation in the colour domain. Whichever separation technique may be employed, the concept is to distinguish between primary radiation (main path) and secondary radiation (reference path).

The relationship between the effect of skinprint volume on radiation in the embodiments disclosed herein has been determined through the use of other techniques, including the use of white light interferometry.

In one exemplary approach, white light interferometry has been used to obtain a surface profile of a skin-print. White light interferometry provides nanometre-accurate three-dimensional surface profile maps of substrates in the nanometre to centimetre surface area range. By obtaining a 3D surface map of the substrate, the volume of the deposited skin-print has been calculated by performing a three-dimensional integration of the volume beneath the surface profile of the deposited skin-print in order to obtain a volume of deposited skin-print, typically in nanolitres.

In this way, the Applicant has identified that there is a close linear correlation between the results of the optical analysis technique of the embodiments described herein and the volume of the skinprint as obtained using the white light interferometry technique.

In particular, the Applicant has identified, over a wide population of skinprints, a linear correlation between the extent to which electromagnetic radiation is coupled by an untreated latent residual skin-print into or out of an optical waveguide and the volume of the said skin-print as determined by white light interferometry.

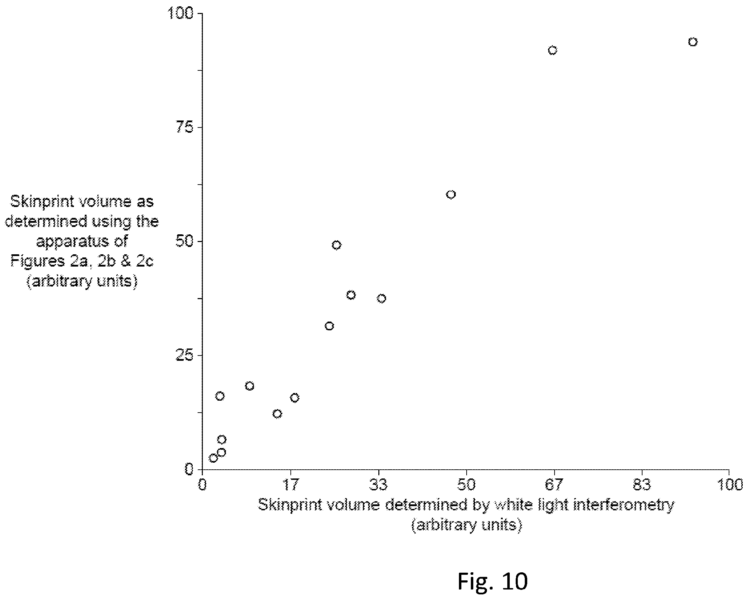

Figure 10 shows data for skinprint volume obtained using the apparatus illustrated in Figures 9a, 9b and 9c plotted against data for skinprint volume determined using the white light interferometry technique.

The data shown in Figure 10 for skinprint volume (obtained using the apparatus illustrated in Figures 9a, 9b and 9c) is a function of the primary radiation 70 that reaches the photodetector 50 through being coupled into the waveguide by the skinprint and includes a compensation factor related to the proportion of secondary radiation losses between the secondary electromagnetic radiation source 80 and the photodetector 50.

The skilled person will appreciate that aspects of different embodiments described herein may be combined, including in ways not explicitly recited. For example, in the case of the seventh embodiment, it may be appropriate to use two photodiodes, in the manner of embodiments 2, 4, 5 and 6. Similarly, it may be appropriate to use a secondary electromagnetic radiation source in any of embodiments 1 to 6.

The skilled person will understand that refraction necessarily occurs when electromagnetic radiation passes between materials having different refractive indices (unless, of course, the difference of refractive indices is such as to result in total internal reflection). For the sake of clarity only, refraction is not shown in the schematic representations of Figures 1a to 8b.

The angle of incidence, θᵢ, at which the primary electromagnetic radiation 70 reaches the waveguide interface 18 is necessarily greater than the angle of incidence, θᵢ₂, at which the secondary electromagnetic radiation 75 reaches the waveguide interface 18. The exact values for θᵢ and θᵢ₂ will depend, among other things, on the refractive indices of the material used for the translucent waveguide 10 and the material (e.g. ambient air) on adjacent the waveguide interface 18 of the translucent waveguide 10.

Where total internal reflection of the primary electromagnetic radiation 70 having the angle of incidence, θᵢ, occurs at the waveguide interface 18 it reflects at an angle of reflection, θᵣ.

While the schematic Figures show the electromagnetic radiation taking only a single path, as the skilled person will readily appreciate, the path of the radiation will diverge. The single lines shown in the Figures are intended to represent the average path of the radiation and for clarity the divergence of radiation from the average path is not shown.

The primary and/or secondary electromagnetic radiation sources may be a source of visible spectrum radiation. The primary and/or secondary light source may be an LED, a filament bulb, a laser, a fluorescent bulb, or any other suitable source of electromagnetic radiation.

The primary and/or secondary electromagnetic radiation may be broad spectrum or narrow spectrum radiation. Potentially, it may be two non-contiguous ranges of narrow spectrum radiation. In some embodiments, the primary and secondary electromagnetic radiation may have the same properties (e.g. wavelength); in other embodiments the primary and secondary electromagnetic radiation may be selected to have different properties (e.g. wavelength).

While the specific embodiments employ one or more photodiodes as electromagnetic radiation detector(s), any appropriate electromagnetic radiation detector(s) may be used. Choice of electromagnetic radiation detector may be dependent, among other things, on the electromagnetic radiation source. Possible electromagnetic radiation detectors include: a photodiode; a phototransistor; a CCD sensor; and a light dependent resistor.

It may be appropriate to use a camera and/or a photomultiplier instead of or in addition to the electromagnetic radiation detector(s) shown in the specific embodiments. In particular, a camera may be used to provide an image of the electromagnetic radiation which may be compared to a database of such images for confirming the identity of a skinprint subject.

While the term skinprint is used throughout this specification, it will be appreciated that the most frequently used form of skinprint is currently the fingerprint (which includes the thumb-print). Nevertheless, other skinprints may be appropriate, such as (but not limited to) a hand-print, a toe-print, a footprint or an ear-print.

The translucent waveguide of any of the embodiments may be any translucent having appropriate properties of transmissivity of electromagnetic radiation of the appropriate wavelengths. The translucent waveguide may be transparent. It may be a glass slide or a plastic slide. An off the shelf slide may be particularly appropriate in embodiments where the translucent waveguide is intended to be a consumable item whereby a new translucent waveguide is employed for each test. If a plastic slide is employed, it may be produced by injection moulding and optionally it may be plasma treated to obtain desirable waveguide properties.

### Terminology

In the context of the present disclosure, the terms "skinprint" (also "skin-print") and "deposited skinprint" are used to refer to a skinprint that is latent and/or residual. That is to say the skinprint is what is left behind on a surface once the human skin, from which the skinprint is derived, has been removed. In this way, the volume of residual skinprint deposited is fixed once the print deposition is complete. The term skinprint is independent of the size and geometry of the substrate and/or the typical area of contact. As the skilled person would readily appreciate, for the purposes of chemical analysis of the skinprint it is important that the skinprint is not diluted for example by such material as ink which, while may assist for the purpose of visualising the skinprint, may compromise chemical analysis.

## Claims

1. A method of determining volume of a deposited residual skinprint, the method comprising:
using an apparatus comprising: a primary electromagnetic radiation source configured to emit visible spectrum electromagnetic radiation; an electromagnetic radiation detector; and a translucent waveguide comprising a first surface providing a waveguide interface coincident with a skinprint receiving region;
transmitting primary electromagnetic radiation from the primary electromagnetic radiation source towards the waveguide interface at an angle of incidence relative to and on a first side of a normal line that is perpendicular to the waveguide interface, such that:
(a) where the waveguide interface interfaces directly with ambient, the primary electromagnetic radiation incident on the waveguide interface undergoes total internal reflection so as to reflect in the waveguide interface at an angle of reflection relative to and on a second side of the normal line opposite the first side; and
(b) where a deposited skinprint is present on the skinprint receiving region such that the waveguide interface interfaces with the skinprint and the skinprint interfaces with ambient, at least a portion of the primary electromagnetic radiation incident on the waveguide interface is caused by the skinprint to be transmitted through the waveguide interface;
using the electromagnetic radiation detector to determine a primary output value being an amount of primary electromagnetic radiation transmitted through the waveguide interface or reflected by the waveguide interface; and
using calibration data that provides correspondence between the primary output value and the volume of skinprint on the substrate so as to provide a value for the volume of the deposited skinprint.

2. The method of claim 1 wherein the calibration data comprises a primary output value for each of a range of volume data obtained independently for a population of calibration skinprints; wherein optionally:
the volume data is obtained for each member of the population of calibration skinprints via a technique comprising white light interferometry analysis of the topography of each of the calibration skinprints to determine volume for each skinprint; and wherein optionally:
the white light interferometry analysis involves data processing to subtract from the topography of the measured area a topography of the translucent waveguide.

3. The method of claim 1 wherein the portion of the primary electromagnetic radiation that is transmitted through the waveguide interface is transmitted in a direction such as to enter the translucent waveguide; wherein optionally:
the translucent waveguide comprises an output grating coupler and such that the portion of primary electromagnetic radiation that is transmitted through the waveguide interface and propagates through the translucent waveguide by total internal reflection exits the translucent waveguide via the output grating.

4. The method of any of claims 1 to 3 wherein the portion of the primary electromagnetic radiation that is transmitted through the waveguide interface is transmitted in a direction such as to exit the translucent waveguide; wherein optionally:
the step of transmitting primary electromagnetic radiation from the primary electromagnetic radiation source towards the waveguide interface is preceded by transmitting the primary electromagnetic radiation into the translucent waveguide at an angle so as to cause the primary electromagnetic radiation to propagate through the translucent waveguide by total internal reflection towards the waveguide interface; wherein optionally:
the translucent waveguide comprises an input grating coupler and wherein the step of transmitting the primary electromagnetic radiation into the translucent waveguide comprises transmitting the primary electromagnetic radiation towards the input grating coupler so as to enter the translucent waveguide; wherein optionally:
the translucent waveguide comprises an output grating coupler and wherein primary electromagnetic radiation that propagates within the translucent waveguide without transmitting through the waveguide interface exits the translucent waveguide via the output grating.

5. The method of any preceding claim wherein the step of using the electromagnetic radiation detector to determine the amount of electromagnetic radiation transmitted through the waveguide interface and/or reflected by the waveguide interface involves one of the following:
using the electromagnetic radiation detector to detect an amount of electromagnetic radiation that exits the translucent waveguide having passed through the waveguide interface;
using the electromagnetic radiation detector to detect an amount of electromagnetic radiation that exits the translucent waveguide without having passed through the waveguide interface.

6. The method of any preceding claim wherein the apparatus further comprises a secondary electromagnetic radiation source configured to provide secondary electromagnetic radiation directed into the waveguide and the method further comprises detecting the secondary electromagnetic radiation to provide data regarding optical properties of the waveguide independent of a skinprint; wherein optionally:
the primary output value includes a compensation factor calculated from the data regarding optical properties of the waveguide; and wherein optionally:
the calibration data that provides correspondence between the primary output value and the volume of skinprint on the substrate is also subject to the compensation factor.

7. The method of claim 6 wherein the secondary electromagnetic radiation is transmitted into the translucent waveguide at an angle such that the secondary electromagnetic radiation is transmitted through the waveguide interface without undergoing total internal reflection at the waveguide interface; wherein optionally the method further comprises:
pulsing either or both of the first electromagnetic radiation source and the second electromagnetic radiation source.

8. The method of any preceding claim wherein the primary electromagnetic radiation source is configured to produce broad spectrum electromagnetic radiation and wherein a calculation unit is configured to compare a spectrum of the electromagnetic radiation detected by the electromagnetic radiation detector with a spectrum of the electromagnetic radiation of the electromagnetic radiation source,

9. The method of claim 8, further comprising:
identifying spectral differences at wavelengths indicative of the presence of one or more particular constituents of human sweat to provide an indication of their potential presence.

10. The method of any of claims 1 to 8 wherein the primary electromagnetic radiation is of a specific wavelength selected for its sensitivity to one or more constituents that may be present in a skinprint.

11. The method of any preceding claim wherein the skinprint receiving region comprises a colour-sensitive coating that changes colour in response to the presence of one or more substances, wherein the method comprises using the electromagnetic radiation detector to detect for the presence of colour.

12. The method of any preceding claim further comprising capturing an image of the skinprint region and, optionally, wherein the method comprises comparing the captured image of the skinprint receiving region with entries in a database of captured skinprint images; and wherein optionally:
the method further comprises seeking a match between the captured image of the skinprint receiving region and one of the entries in the database of captured skinprint images in order to provide an indication of identity of the skinprint.

13. The method of any preceding claim wherein the electromagnetic radiation detector comprises a primary electromagnetic radiation detector and a secondary electromagnetic radiation detector and wherein either:
(a) the primary electromagnetic radiation detector is used to detect electromagnetic radiation transmitted through the waveguide interface; and the secondary electromagnetic radiation detector is used to detect electromagnetic radiation reflected by the waveguide interface; or
(b) the secondary electromagnetic radiation detector is configured to provide a measure of strength of electromagnetic radiation emitted by the primary electromagnetic radiation source.

14. The method of any proceeding claim further comprising providing a binary output to indicate whether a predefined threshold of skinprint volume is detected; wherein optionally:
the predefined threshold of skinprint volume is chosen as a minimum volume for which a subsequent chemical analysis is reliably performable.

15. A device comprising: a primary electromagnetic radiation source; an electromagnetic radiation detector; and a translucent waveguide comprising a first surface providing a waveguide interface coincident with a skinprint receiving region,
wherein the device is configured to perform the method of any preceding claim; and optionally any one or more of:
the device is configured to provide an output measured in unit mass of analyte per unit volume of skinprint;
the device is portable; and
the device comprises a skinprint receiving region having an area smaller than an area of an average adult skinprint.

## Patentansprüche

1. Verfahren zum Bestimmen des Volumens eines abgelagerten restlichen Hautabdrucks, wobei das Verfahren umfasst:
Verwenden einer Vorrichtung, die umfasst: eine primäre elektromagnetische Strahlungsquelle, die so konfiguriert ist, dass sie elektromagnetische Strahlung im sichtbaren Spektrum emittiert; einen Detektor für elektromagnetische Strahlung; und einen lichtdurchlässigen Wellenleiter, der eine erste Oberfläche umfasst, die eine Wellenleiter-Schnittstelle bereitstellt, die mit einem Hautabdruck-Empfangsbereich übereinstimmt;
Übertragen der primären elektromagnetischen Strahlung von der primären elektromagnetischen Strahlungsquelle zu der Wellenleiter-Schnittstelle unter einem Einfallswinkel relativ zu und auf einer ersten Seite einer Normalen, die senkrecht zu der Wellenleiter-Schnittstelle steht, so dass:
(a) dort wo die Wellenleiter-Schnittstelle direkt mit der Umgebung in Kontakt steht, die auf die Wellenleiter-Schnittstelle einfallende primäre elektromagnetische Strahlung eine Totalreflexion erfährt, so dass sie in der Wellenleiter-Schnittstelle in einem Reflexionswinkel relativ zu und auf einer zweiten Seite der Normalen gegenüber der ersten Seite reflektiert wird; und
(b) dort wo ein abgelagerter Hautabdruck auf dem Hautabdruck-Empfangsbereich vorhanden ist, so dass die Wellenleiter-Schnittstelle mit dem Hautabdruck in Kontakt steht und der Hautabdruck mit der Umgebung in Kontakt steht, mindestens ein Teil der auf die Wellenleiter-Schnittstelle einfallenden primären elektromagnetischen Strahlung durch den Hautabdruck dazu gebracht wird, durch die Wellenleiter-Schnittstelle hindurch übertragen zu werden;
Verwenden des Detektors für elektromagnetische Strahlung, um einen primären Ausgabewert zu bestimmen, der eine Menge an primärer elektromagnetischer Strahlung ist, die durch die Wellenleiter-Schnittstelle übertragen oder von der Wellenleiter-Schnittstelle reflektiert wird; und
Verwenden von Kalibrierungsdaten, die eine Entsprechung zwischen dem primären Ausgabewert und dem Volumen des Hautabdrucks auf dem Substrat bereitstellen, um einen Wert für das Volumen des abgelagerten Hautabdrucks bereitzustellen.

2. Verfahren nach Anspruch 1, wobei die Kalibrierungsdaten einen Primärausgabewert für jeden eines Bereichs von Volumendaten umfassen, die unabhängig für eine Population von Kalibrierungshautabdrücken erhalten werden, wobei optional:
die Volumendaten für jedes Mitglied der Population von Kalibrierungshautabdrücken mittels einer Technik erhalten werden, die eine Weißlichtinterferometrieanalyse der Topografie jedes der Kalibrierungshautabdrücke umfasst, um das Volumen für jeden Hautabdruck zu bestimmen, und wobei optional:
die Weißlichtinterferometrieanalyse eine Datenverarbeitung umfasst, um von der Topografie des gemessenen Bereichs eine Topografie des lichtdurchlässigen Wellenleiters zu subtrahieren.

3. Verfahren nach Anspruch 1, wobei der Teil der primären elektromagnetischen Strahlung, der durch die Wellenleiter-Schnittstelle übertragen wird, in einer Richtung übertragen wird, so dass er in den lichtdurchlässigen Wellenleiter eintritt, wobei optional:
der lichtdurchlässige Wellenleiter einen Ausgabegitterkoppler umfasst und so beschaffen ist, dass der Teil der primären elektromagnetischen Strahlung, der durch die Wellenleiter-Schnittstelle übertragen wird und sich durch den lichtdurchlässigen Wellenleiter mittels Totalreflexion ausbreitet, den lichtdurchlässigen Wellenleiter über das Ausgabegitter verlässt.

4. Verfahren nach einem der Ansprüche 1 bis 3, wobei der Teil der primären elektromagnetischen Strahlung, der durch die Wellenleiter-Schnittstelle übertragen wird, in einer Richtung übertragen wird, so dass er den lichtdurchlässigen Wellenleiter verlässt, wobei optional:
dem Schritt des Übertragens der primären elektromagnetischen Strahlung von der primären elektromagnetischen Strahlungsquelle zur Wellenleiter-Schnittstelle das Übertragen der primären elektromagnetischen Strahlung in den lichtdurchlässigen Wellenleiter in einem Winkel vorausgeht, so dass die primäre elektromagnetische Strahlung durch Totalreflexion durch den lichtdurchlässigen Wellenleiter zur Wellenleiter-Schnittstelle propagiert, wobei optional:
der lichtdurchlässige Wellenleiter einen Eingabegitterkoppler umfasst und wobei der Schritt des Übertragens der primären elektromagnetischen Strahlung in den lichtdurchlässigen Wellenleiter das Übertragen der primären elektromagnetischen Strahlung in Richtung des Eingabegitterkopplers umfasst, so dass sie in den lichtdurchlässigen Wellenleiter eintritt, wobei optional:
der lichtdurchlässige Wellenleiter einen Ausgabegitterkoppler umfasst und wobei die primäre elektromagnetische Strahlung, die sich innerhalb des lichtdurchlässigen Wellenleiters, ohne Übertragung durch die Wellenleiter-Schnittstelle, ausbreitet, den lichtdurchlässigen Wellenleiter über das Ausgabegitter verlässt.

5. Verfahren nach einem der vorhergehenden Ansprüche, wobei der Schritt des Verwendens des Detektors für elektromagnetische Strahlung zum Bestimmen der Menge an elektromagnetischer Strahlung, die durch die Wellenleiter-Schnittstelle übertragen und/oder von der Wellenleiter-Schnittstelle reflektiert wird, eines der folgenden umfasst:
Verwenden des Detektors für elektromagnetische Strahlung, um eine Menge an elektromagnetischer Strahlung zu erfassen, die den lichtdurchlässigen Wellenleiter verlässt, nachdem sie die Wellenleiter-Schnittstelle passiert hat;
Verwenden des Detektors für elektromagnetische Strahlung, um eine Menge an elektromagnetischer Strahlung zu erfassen, die den lichtdurchlässigen Wellenleiter verlässt, ohne die Wellenleiter-Schnittstelle passiert zu haben.

6. Verfahren nach einem der vorhergehenden Ansprüche, wobei die Vorrichtung ferner eine sekundäre elektromagnetische Strahlungsquelle umfasst, die so konfiguriert ist, dass sie sekundäre elektromagnetische Strahlung bereitstellt, die in den Wellenleiter geleitet wird, und das Verfahren ferner das Erfassen der sekundären elektromagnetischen Strahlung umfasst, um Daten bezüglich der optischen Eigenschaften des Wellenleiters unabhängig von einem Hautabdruck bereitzustellen, wobei optional:
der primäre Ausgabewert einen Kompensationsfaktor umfasst, der aus den Daten über die optischen Eigenschaften des Wellenleiters berechnet wird, und wobei optional:
die Kalibrierungsdaten, die eine Entsprechung zwischen dem primären Ausgabewert und dem Volumen des Hautabdrucks auf dem Substrat bereitstellen, ebenfalls dem Kompensationsfaktor unterliegen.

7. Verfahren nach Anspruch 6, wobei die sekundäre elektromagnetische Strahlung in einem solchen Winkel in den lichtdurchlässigen Wellenleiter eingestrahlt wird, dass die sekundäre elektromagnetische Strahlung durch die Wellenleiter-Schnittstelle hindurchgeht, ohne an der Wellenleiter-Schnittstelle eine Totalreflexion zu erfahren, wobei das Verfahren optional ferner umfasst:
Pulsieren entweder der ersten elektromagnetischen Strahlungsquelle oder der zweiten elektromagnetischen Strahlungsquelle oder beider.

8. Verfahren nach einem der vorhergehenden Ansprüche, wobei die primäre elektromagnetische Strahlungsquelle so konfiguriert ist, dass sie elektromagnetische Strahlung mit breitem Spektrum erzeugt, und wobei eine Berechnungseinheit so konfiguriert ist, dass sie ein Spektrum der vom Detektor für elektromagnetische Strahlung erfassten elektromagnetischen Strahlung mit einem Spektrum der elektromagnetischen Strahlung der elektromagnetischen Strahlungsquelle vergleicht.

9. Verfahren nach Anspruch 8, das ferner umfasst
Identifizieren von Spektralunterschieden bei Wellenlängen, die auf das Vorhandensein eines oder mehrerer bestimmter Bestandteile von menschlichem Schweiß hinweisen, um einen Hinweis auf deren potenzielles Vorhandensein zu liefern.

10. Verfahren nach einem der Ansprüche 1 bis 8, wobei die primäre elektromagnetische Strahlung eine bestimmte Wellenlänge aufweist, die aufgrund ihrer Empfindlichkeit gegenüber einem oder mehreren Bestandteilen ausgewählt ist, die in einem Hautabdruck vorhanden sein können.

11. Verfahren nach einem der vorhergehenden Ansprüche, wobei der Hautabdruck-Empfangsbereich eine farbsensitive Beschichtung umfasst, die ihre Farbe in Reaktion auf das Vorhandensein einer oder mehrerer Substanzen ändert, wobei das Verfahren die Verwendung des Detektors für elektromagnetische Strahlung umfasst, um das Vorhandensein von Farbe zu erfassen.

12. Verfahren nach einem der vorhergehenden Ansprüche, das ferner das Aufnehmen eines Bildes des Hautabdruckbereichs umfasst, wobei das Verfahren optional ferner das Vergleichen des aufgenommenen Bildes des Hautabdruck-Empfangsbereichs mit Einträgen in einer Datenbank mit aufgenommenen Hautabdruckbildern umfasst und optional:
das Verfahren ferner das Suchen einer Übereinstimmung zwischen dem aufgenommenen Bild des Hautabdruck-Empfangsbereichs und einem der Einträge in der Datenbank mit aufgenommenen Hautabdruckbildern umfasst, um einen Hinweis auf die Identität des Hautabdrucks zu liefern.

13. Verfahren nach einem der vorhergehenden Ansprüche, wobei der Detektor für elektromagnetische Strahlung einen primären Detektor für elektromagnetische Strahlung und einen sekundären Detektor für elektromagnetische Strahlung umfasst und wobei entweder:
(a) der primäre Detektor für elektromagnetische Strahlung zum Erfassen von durch die Wellenleiter-Schnittstelle übertragener elektromagnetischer Strahlung verwendet wird, und der sekundäre Detektor für elektromagnetische Strahlung zum Erfassen von durch die Wellenleiter-Schnittstelle reflektierter elektromagnetischer Strahlung verwendet wird, oder
(b) der sekundäre Detektor für elektromagnetische Strahlung so konfiguriert ist, dass er ein Maß für die Stärke der von der primären Quelle für elektromagnetische Strahlung emittierten elektromagnetischen Strahlung liefert.

14. Verfahren nach einem der vorhergehenden Ansprüche, das ferner das Bereitstellen einer binären Ausgabe umfasst, um anzuzeigen, ob ein vordefinierter Schwellenwert für das Hautabdruckvolumen erfasst wurde, wobei optional:
der vordefinierte Schwellenwert für das Hautabdruckvolumen als Mindestvolumen gewählt wird, für das eine nachfolgende chemische Analyse zuverlässig durchführbar ist.

15. Vorrichtung, umfassend: eine primäre elektromagnetische Strahlungsquelle, einen Detektor für elektromagnetische Strahlung und einen lichtdurchlässigen Wellenleiter, der eine erste Oberfläche umfasst, die eine Wellenleiter-Schnittstelle bereitstellt, die mit einem Hautabdruck-Empfangsbereich übereinstimmt,
wobei die Vorrichtung so konfiguriert ist, dass sie das Verfahren eines vorhergehenden Anspruchs durchführt, und optional eines oder mehrere der folgenden Merkmale aufweist:
die Vorrichtung ist so konfiguriert, dass sie einen Ausgabewert liefert, der in Masseeinheiten des Analyten pro Volumeneinheit des Hautabdrucks gemessen wird,
die Vorrichtung ist tragbar, und
die Vorrichtung umfasst einen Hautabdruck-Empfangsbereich, dessen Fläche kleiner ist als die Fläche eines durchschnittlichen Hautabdrucks eines Erwachsenen.

## Revendications

1. Procédé de détermination du volume d'une empreinte cutanée résiduelle déposée, le procédé comportant :
l'utilisation d'un appareil comportant : une source de rayonnement électromagnétique primaire configurée pour émettre un rayonnement électromagnétique à spectre visible ; un détecteur de rayonnement électromagnétique ; et un guide d'ondes translucide comportant une première surface fournissant une interface de guide d'ondes coïncidant avec une région de réception d'empreinte cutanée ;
la transmission d'un rayonnement électromagnétique primaire à partir de la source de rayonnement électromagnétique primaire vers l'interface de guide d'ondes à un angle d'incidence par rapport à et sur un premier côté d'une ligne normale qui est perpendiculaire à l'interface de guide d'ondes, de sorte que :
(a) lorsque l'interface de guide d'ondes s'interface directement avec l'environnement, le rayonnement électromagnétique primaire incident sur l'interface de guide d'ondes subit une réflexion interne totale de manière à se réfléchir dans l'interface de guide d'ondes à un angle de réflexion par rapport à et sur un deuxième côté de la ligne normale opposé au premier côté ; et
(b) lorsqu'une empreinte cutanée déposée est présente sur la région de réception d'empreinte cutanée de sorte que l'interface de guide d'ondes s'interface avec l'empreinte cutanée et les interfaces d'empreinte cutanée avec l'environnement, au moins une partie du rayonnement électromagnétique primaire incident sur l'interface de guide d'ondes est causée par l'empreinte cutanée à transmettre par l'intermédiaire de l'interface de guide d'ondes ;
l'utilisation du détecteur de rayonnement électromagnétique pour déterminer une valeur de sortie primaire qui est une quantité de rayonnement électromagnétique primaire transmis par l'intermédiaire de l'interface de guide d'ondes ou réfléchi par l'interface de guide d'ondes ; et
l'utilisation de données d'étalonnage qui fournissent une correspondance entre la valeur de sortie primaire et le volume d'empreinte cutanée sur le substrat de manière à fournir une valeur pour le volume de l'empreinte cutanée déposée.

2. Procédé selon la revendication 1 dans lequel les données d'étalonnage comportent une valeur de sortie primaire pour chacune d'une plage de données de volume obtenues indépendamment pour une population d'empreintes cutanées d'étalonnage ; dans lequel facultativement :
les données de volume sont obtenues pour chaque membre de la population d'empreintes cutanées d'étalonnage via une technique comportant une analyse par interférométrie en lumière blanche de la topographie de chacune des empreintes cutanées d'étalonnage pour déterminer le volume pour chaque empreinte cutanée ; et dans lequel facultativement :
l'analyse par interférométrie en lumière blanche implique un traitement de données pour soustraire de la topographie de la zone mesurée une topographie du guide d'ondes translucide.

3. Procédé selon la revendication 1 dans lequel la partie du rayonnement électromagnétique primaire qui est transmise par l'intermédiaire de l'interface de guide d'ondes est transmise dans une direction telle que pour entrer dans le guide d'ondes translucide ; dans lequel facultativement :
le guide d'ondes translucide comporte un coupleur de grille de sortie et de sorte que la partie de rayonnement électromagnétique primaire qui est transmise par l'intermédiaire de l'interface de guide d'ondes et se propage par l'intermédiaire du guide d'ondes translucide par réflexion interne totale sort du guide d'ondes translucide via la grille de sortie.

4. Procédé selon l'une quelconque des revendications 1 à 3 dans lequel la partie du rayonnement électromagnétique primaire qui est transmise par l'intermédiaire de l'interface de guide d'ondes est transmise dans une direction telle que pour sortir du guide d'ondes translucide ; dans lequel facultativement :
l'étape de transmission d'un rayonnement électromagnétique primaire à partir de la source de rayonnement électromagnétique primaire vers l'interface de guide d'ondes est précédée de la transmission du rayonnement électromagnétique primaire dans le guide d'ondes translucide à un angle de manière à amener le rayonnement électromagnétique primaire à se propager par l'intermédiaire du guide d'ondes translucide par réflexion interne totale vers l'interface de guide d'ondes ; dans lequel facultativement :
le guide d'ondes translucide comporte un coupleur de grille d'entrée et dans lequel l'étape de transmission du rayonnement électromagnétique primaire dans le guide d'ondes translucide comporte la transmission du rayonnement électromagnétique primaire vers le coupleur de grille d'entrée de manière à entrer dans le guide d'ondes translucide ; dans lequel facultativement :
le guide d'ondes translucide comporte un coupleur de grille de sortie et dans lequel le rayonnement électromagnétique primaire qui se propage à l'intérieur du guide d'ondes translucide sans transmission par l'intermédiaire de l'interface de guide d'ondes sort du guide d'ondes translucide via la grille de sortie.

5. Procédé selon une quelconque revendication précédente dans lequel l'étape d'utilisation du détecteur de rayonnement électromagnétique pour déterminer la quantité de rayonnement électromagnétique transmis par l'intermédiaire de l'interface de guide d'ondes et/ou réfléchi par l'interface de guide d'ondes implique l'un de ce qui suit :
l'utilisation du détecteur de rayonnement électromagnétique pour détecter une quantité de rayonnement électromagnétique qui sort du guide d'ondes translucide ayant traversé l'interface de guide d'ondes ;
l'utilisation du détecteur de rayonnement électromagnétique pour détecter une quantité de rayonnement électromagnétique qui sort du guide d'ondes translucide sans avoir traversé l'interface de guide d'ondes.

6. Procédé selon une quelconque revendication précédente dans lequel l'appareil comporte en outre une source de rayonnement électromagnétique secondaire configurée pour fournir un rayonnement électromagnétique secondaire dirigé dans le guide d'ondes et le procédé comporte en outre la détection du rayonnement électromagnétique secondaire pour fournir des données concernant les propriétés optiques du guide d'ondes indépendamment d'une empreinte cutanée ; dans lequel facultativement :
la valeur de sortie primaire inclut un facteur de compensation calculé à partir des données concernant les propriétés optiques du guide d'ondes ; et dans lequel facultativement :
les données d'étalonnage qui fournissent une correspondance entre la valeur de sortie primaire et le volume d'empreinte cutanée sur le substrat sont également soumises au facteur de compensation.

7. Procédé selon la revendication 6 dans lequel le rayonnement électromagnétique secondaire est transmis dans le guide d'ondes translucide à un angle tel que le rayonnement électromagnétique secondaire est transmis par l'intermédiaire de l'interface de guide d'ondes sans subir de réflexion interne totale au niveau de l'interface de guide d'ondes ; dans lequel facultativement le procédé comporte en outre :
l'impulsion de l'une ou des deux de la première source de rayonnement électromagnétique et de la deuxième source de rayonnement électromagnétique.

8. Procédé selon une quelconque revendication précédente dans lequel la source de rayonnement électromagnétique primaire est configurée pour produire un rayonnement électromagnétique à large spectre et dans lequel une unité de calcul est configurée pour comparer un spectre du rayonnement électromagnétique détecté par le détecteur de rayonnement électromagnétique avec un spectre du rayonnement électromagnétique de la source de rayonnement électromagnétique.

9. Procédé selon la revendication 8, comportant en outre :
l'identification de différences spectrales à des longueurs d'onde indiquant la présence d'un ou plusieurs constituants particuliers de la sueur humaine pour fournir une indication de leur présence potentielle.

10. Procédé selon l'une quelconque des revendications 1 à 8 dans lequel le rayonnement électromagnétique primaire est d'une longueur d'onde spécifique sélectionnée pour sa sensibilité à un ou plusieurs constituants qui peuvent être présents dans une empreinte cutanée.

11. Procédé selon une quelconque revendication précédente dans lequel la région réceptrice d'empreinte cutanée comporte un revêtement sensible à la couleur qui change de couleur en réponse à la présence d'une ou plusieurs substances, dans lequel le procédé comporte l'utilisation du détecteur de rayonnement électromagnétique pour détecter la présence de couleur.

12. Procédé selon une quelconque revendication précédente comportant en outre la capture d'une image de la région d'empreinte cutanée et, facultativement, dans lequel le procédé comporte la comparaison de l'image capturée de la région de réception d'empreinte cutanée avec des entrées dans une base de données d'images d'empreinte cutanée capturées ; et dans lequel facultativement :
le procédé comporte en outre la recherche d'une correspondance entre l'image capturée de la région de réception d'empreinte cutanée et l'une des entrées dans la base de données d'images d'empreinte cutanée capturées afin de fournir une indication d'identité de l'empreinte cutanée.

13. Procédé selon une quelconque revendication précédente dans lequel le détecteur de rayonnement électromagnétique comporte un détecteur de rayonnement électromagnétique primaire et un détecteur de rayonnement électromagnétique secondaire et dans lequel soit :
(a) le détecteur de rayonnement électromagnétique primaire est utilisé pour détecter un rayonnement électromagnétique transmis par l'intermédiaire de l'interface du guide d'ondes ; et le détecteur de rayonnement électromagnétique secondaire est utilisé pour détecter un rayonnement électromagnétique réfléchi par l'interface du guide d'ondes ; soit
(b) le détecteur de rayonnement électromagnétique secondaire est configuré pour fournir une mesure de l'intensité de rayonnement électromagnétique émis par la source de rayonnement électromagnétique primaire.

14. Procédé selon une quelconque revendication précédente comportant en outre la fourniture d'une sortie binaire pour indiquer si un seuil prédéfini de volume d'empreinte cutanée est détecté ; dans lequel facultativement :
le seuil prédéfini de volume d'empreinte cutanée est choisi comme volume minimal pour lequel une analyse chimique ultérieure est réalisable de manière fiable.

15. Dispositif comportant : une source de rayonnement électromagnétique primaire ; un détecteur de rayonnement électromagnétique ; et un guide d'ondes translucide comportant une première surface fournissant une interface de guide d'ondes coïncidant avec une région de réception d'empreinte cutanée,
dans lequel le dispositif est configuré pour réaliser le procédé selon une quelconque revendication précédente ; et facultativement l'un ou plusieurs de :
le dispositif est configuré pour fournir une sortie mesurée en masse unitaire d'analyte par volume unitaire d'empreinte cutanée ;
le dispositif est portable ; et
le dispositif comporte une région de réception d'empreinte cutanée ayant une superficie plus petite qu'une superficie d'empreinte cutanée moyenne d'adulte.
